# EUROPEAN PATENT SPECIFICATION

(11) **EP 3 625 331 B1**
(45) Date of publication and mention of the grant of the patent: **10.07.2024**
(21) Application number: 18802136.4
(22) Date of filing: 18.05.2018
(51) Int. Cl.: C12N 5/074, C12N 5/0789, C12N 5/071, G01N 33/50, A01K 67/027

(54) **SYSTEMS AND METHODS FOR GROWTH OF INTESTINAL CELLS**
SYSTEME UND VERFAHREN ZUM WACHSTUM VON DARMZELLEN
SYSTÈMES ET MÉTHODES DE CROISSANCE DE CELLULES INTESTINALES

(30) Priority: 19.05.2017 US 201762508721 P
(43) Date of publication of application: 25.03.2020
(73) Proprietor: Cedars-Sinai Medical Center, Los Angeles, CA 90048 (US)
(72) Inventor: BARRETT, Robert, Los Angeles, California 90036 (US); SVENDSEN, Clive, Pacific Palisades, California 90272 (US); TARGAN, Stephan R., Santa Monica, California 90402 (US); WORKMAN, Michael, Santa Monica, California 90402 (US); SAREEN, Dhruv, Porter Ranch, California 91326 (US)
(74) Representative: Hutter, Anton
(86) International application number: PCT/US2018/033498
(87) International publication number: WO 2018/213773

(56) References cited:
- WO-A1-2015/188131
- WO-A1-2016/061464
- WO-A1-2016/093222
- WO-A1-2018/106628
- WO-A2-2017/136462
- US-A1- 2012 196 312
- US-A1- 2013 137 130
- US-A1- 2015 232 810
- KURATNIK ANTON ET AL: "Intestinal organoids as tissue surrogates for toxicological and pharmacological studies", BIOCHEMICAL PHARMACOLOGY, ELSEVIER, US, vol. 85, no. 12, 25 April 2013 (2013-04-25), pages 1721 - 1726, XP028560680, ISSN: 0006-2952, DOI: 10.1016/J.BCP.2013.04.016
- WORKMAN MICHAEL ET AL: "Intestine-Chip: A New Model to Understand the Role of the Intestinal Epithelium in IBD by Combining Microengineering Technology and IPSC-Derived Human Intestinal Organoids", GASTROENTEROLOGY, vol. 152, no. 5, 1 April 2017 (2017-04-01), XP029979161, ISSN: 0016-5085, DOI: 10.1016/S0016-5085(17)30550-4
- MICHAEL J. WORKMAN ET AL: "Enhanced Utilization of Induced Pluripotent Stem Cell-Derived Human Intestinal Organoids Using Microengineered Chips", CMGH CELLULAR AND MOLECULAR GASTROENTEROLOGY AND HEPATOLOGY, vol. 5, no. 4, 1 January 2018 (2018-01-01), pages 669 - 677.e2, XP055611843, ISSN: 2352-345X, DOI: 10.1016/j.jcmgh.2017.12.008
- BARRETT ET AL.: "Reliable Generation of Induced Pluripotent Stem Cells From Human Lymphoblastoid Cell Lines", STEM CELLS TRANSLATIONAL MEDICINE, vol. 3, no. 12, 8 October 2014 (2014-10-08), pages 1429 - 1434, XP055403729

## Description

### STATEMENT REGARDING FEDERALLY SPONSORED RESEARCH

This invention was made with government support under DK106202 awarded by the National Institutes of Health. The government has certain rights in the invention.

### FIELD OF THE INVENTION

Described herein are methods and compositions related to intestinal and colonic cells, including organoids. These cells can mode human gastrointestinal disorders, such as early onset and very early onset Inflammatory Bowel Disease.

### BACKGROUND

Inflammatory bowel diseases (IBDs) are complex, multifactorial disorders characterized by chronic relapsing intestinal inflammation. Although etiology remains largely unknown, recent research has suggested that genetic factors, environment, microbiota, and immune response are involved in the pathogenesis. Studies examining intestinal epithelial cell function have been severely hampered because primary human intestinal epithelial cells rapidly undergo apoptosis when cultured ex vivo. While adenocarcinoma lines such as Caco2 cells have certainly been useful, a substantial breakthrough in the intestinal epithelial field occurred when it was reported that three dimensional human intestinal "organoids" could be generated from either human biopsy samples. Irrespective of how these organoids were derived, they contained all the intestinal epithelial subtypes, were polarized towards the lumen and could be maintained for prolonged periods of time in a tightly controlled milieu. However, there are substantial technical challenges associated with this technology. Access to the lumen, which is crucial for assessing intestinal permeability, microbial-epithelial interactions and drug absorption is technically challenging, laborious and requires specialized equipment. Coculture with other cell types, such as various immune cell subtypes or endothelial cells, is also difficult given organoids are typically embedded in a three dimensional matrix. These limitations in sample collection and inability to recapitulate the multicellular underpinnings of bowel disease pathogenesis prevents understanding of the incredibly complex genetic diversity behind such diseases. Thus, there is a great need in the art for disease models of bowel disease.

Given that iPSCs can be generated from any individual, iPSC-derived human intestinal organoids (HIOs) and colonic organoids (HCOs) can generated and incorporated into fluidic systems as this facilitates the study of intestinal epithelial and colonic cells from virtually any individual or patient. It also allows for the generation of other patient specific cell types such as macrophages, dendritic cells, neutrophils that could also be incorporated into fluidic devices, including microfluidic chips, to study multicellular interactions.

WO 2015/188131 teaches the generation of iPSCs from lymphoblastoid B cells. US 2017/292116 teaches the generation of intestinal organoids from human embryonic stem cells and iPSCs. Kuratnik, A. et al., 2013. Intestinal organoids as tissue surrogates for toxicological and pharmacological studies. Biochemical pharmacology, 85(12), pp.1721-1726, describes human intestinal organoids as tissue surrogates for toxicological and pharmacological studies derived from pluripotent stem cells. Workman, M. et al., 2017. Intestine-Chip: A New Model to Understand the Role of the Intestinal Epithelium in IBD by Combining Microengineering Technology and IPSC-Derived Human Intestinal Organoids. Gastroenterology, 152(5), pp.S57-S58, describes an intestine-chip as a model to understand the role of intestinal epithelium in IBD by using iPSC-derived human intestinal organoids. Workman, M.J., et al., 2018. Enhanced utilization of induced pluripotent stem cell-derived human intestinal organoids using microengineered chips. Cellular and molecular gastroenterology and hepatology, 5(4), pp.669-677, describes intestinal organoids derived from iPSCs that are incorporated into small microengineered chips. WO 2017/136462 teaches systems and methods for the growth of intestinal cells in microfluidic devices. US 2012/196312 describes culture medium for culturing epithelial stem cells, particularly intestinal and colonic epithelial stem cells. WO 2018/106628 teaches methods for the in vitro differentiation of a precursor cell into definitive endoderm, which may be differentiated into a human colonic organoid.

### SUMMARY OF THE INVENTION

The invention is as defined by the claims and any other aspects, configurations or embodiments set forth herein not falling within the scope of the claims are for information only.

Described herein is a method of generating colonic cells, including generation of definitive endoderm by culturing iPSCs in the presence of Activin A and Wnt3A, differentiation into hindgut by culturing definitive endoderm in the presence of FGF4 and either Wnt3A or CHIR99021, collection of epithelial spheres or epithelial tubes, suspension of epithelial spheres or epithelial tubes in Matrigel, and culturing in the presence of EGF and BMP to generate colonic cells. In other embodiments, the iPSCs are reprogrammed cells from whole or peripheral blood. In other embodiments, the iPSCs are reprogrammed from a non B-Cell, non T-cell component of blood. In other embodiments, the iPSCs are reprogrammed lymphoblastoid B-cell derived induced pluripotent stem cells (LCL-iPSCs). In other embodiments, the iPSCs are reprogrammed cells obtained from a subject afflicted with an inflammatory bowel disease and/or condition. In other embodiments, the inflammatory bowel disease and/or condition is early onset. In other embodiments, the inflammatory bowel disease and/or condition is very early onset. In other embodiments, the colonic cells express one or more of: SATB2, MUC5B, MUC2, INSL5, HOXA13, CKB, FXYD3, and HOXB13.

Further described herein is a composition comprising colonic cells made by a method including generating colonic cells, including generation of definitive endoderm by culturing iPSCs in the presence of Activin A and Wnt3A, differentiation into hindgut by culturing definitive endoderm in the presence of FGF4 and either Wnt3A or CHIR99021, collection of epithelial spheres or epithelial tubes, suspension of epithelial spheres or epithelial tubes in Matrigel, and culturing in the presence of EGF and BMP to generate colonic cells. In other embodiments, the composition is a cryopreserved solution.

Also described herein is a organoid comprising intestinal cells made by a method including generating colonic cells, including generation of definitive endoderm by culturing iPSCs in the presence of Activin A and Wnt3A, differentiation into hindgut by culturing definitive endoderm in the presence of FGF4 and either Wnt3A or CHIR99021, collection of epithelial spheres or epithelial tubes, suspension of epithelial spheres or epithelial tubes in Matrigel, and culturing in the presence of EGF and BMP to generate colonic cells.

Described herein is a method of generating intestinal cells, including generation of definitive endoderm by culturing iPSCs in the presence of Activin A and Wnt3A, differentiation into hindgut by culturing definitive endoderm in the presence of FGF4 and either Wnt3A or CHIR99021, collection of epithelial spheres or epithelial tubes, suspension of epithelial spheres or epithelial tubes in Matrigel, and culturing in the presence of CHIR99021, noggin and EGF to generate intestinal cells. In various embodiments, the iPSCs are reprogrammed cells from whole or peripheral blood. In various embodiments, the iPSCs are reprogrammed from a non B-Cell, non T-cell component of blood. In various embodiments, the iPSCs are reprogrammed lymphoblastoid B-cell derived induced pluripotent stem cells (LCL-iPSCs). In various embodiments, the iPSCs are reprogrammed cells obtained from a subject afflicted with an inflammatory bowel disease and/or condition. In various embodiments, the inflammatory bowel disease and/or condition is early onset. In various embodiments, the inflammatory bowel disease and/or condition is very early onset. In various embodiments, the intestinal cells express one or more of: PDX1, GATA4, and DEFA5.

Also described herein is a composition comprising intestinal cells made by a method of generating intestinal cells, including generation of definitive endoderm by culturing iPSCs in the presence of Activin A and Wnt3A, differentiation into hindgut by culturing definitive endoderm in the presence of FGF4 and either Wnt3A or CHIR99021, collection of epithelial spheres or epithelial tubes, suspension of epithelial spheres or epithelial tubes in Matrigel, and culturing in the presence of CHIR99021, noggin and EGF to generate intestinal cells. In various embodiments, the composition is a cryopreserved solution.

Also described herein is an organoid comprising intestinal cells made by a method of generating intestinal cells, including generation of definitive endoderm by culturing iPSCs in the presence of Activin A and Wnt3A, differentiation into hindgut by culturing definitive endoderm in the presence of FGF4 and either Wnt3A or CHIR99021, collection of epithelial spheres or epithelial tubes, suspension of epithelial spheres or epithelial tubes in Matrigel, and culturing in the presence of CHIR99021, noggin and EGF to generate intestinal cells.

### BRIEF DESCRIPTION OF THE FIGURES

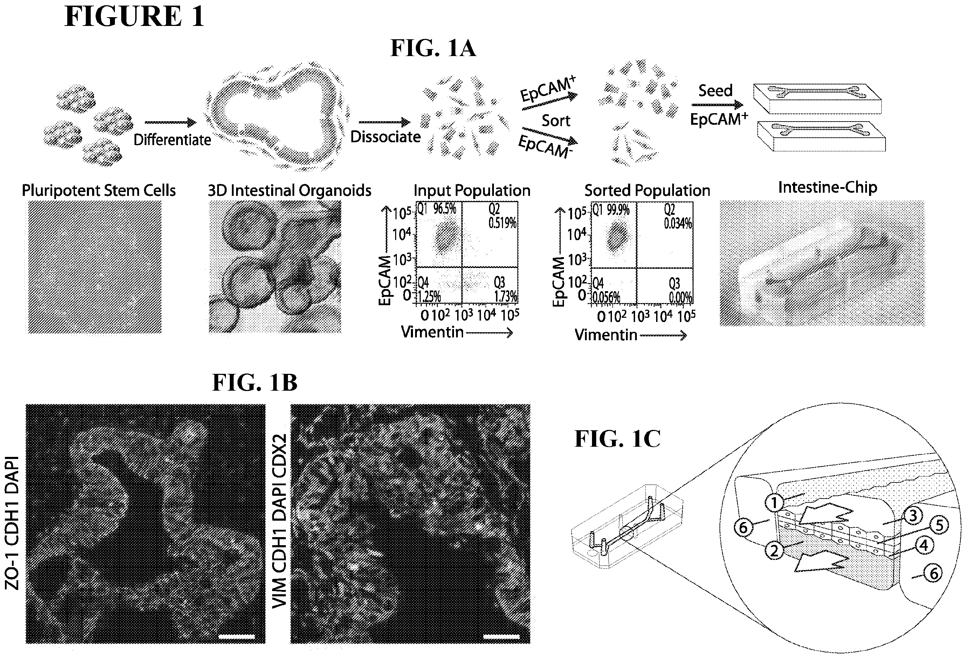

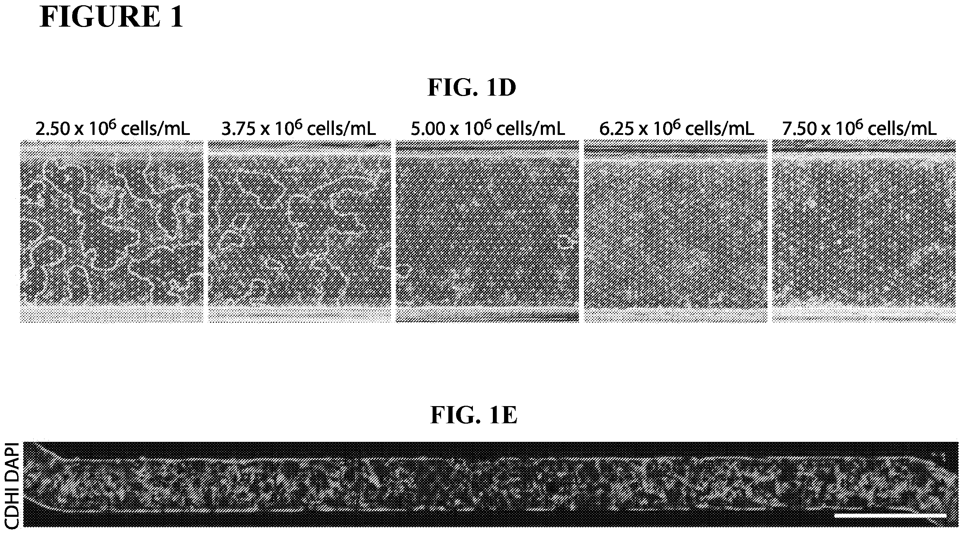

**Figure 1****.** a) Schematic of workflow for incorporation of IPSC-derived HIOs into the Intestine-Chip B) Representative images showing polarized HIOs immunopositive for E-Cadherin (red) and ZO1 (green), and HIOs immunopositive for E-Cadherin (red), CDX2 (gray), and vimentin (green) all counterstained with DAPI (blue). c) Schematic of the small microengineered Chip used in this study. d) HIO-epithelial cells ranging in concentrations from 2.5-7.5×10⁶ cells/ml were seeded into the Chip and representative phase contrast images were obtained after 24hr. Yellow lines denote areas of the Chip not covered by HIO-derived epithelial cells, e) HIO-epithelial cells were seeded into the Chip and E-Cadherin cells+(red) counterstained with DAPI (blue) were imaged three days later.

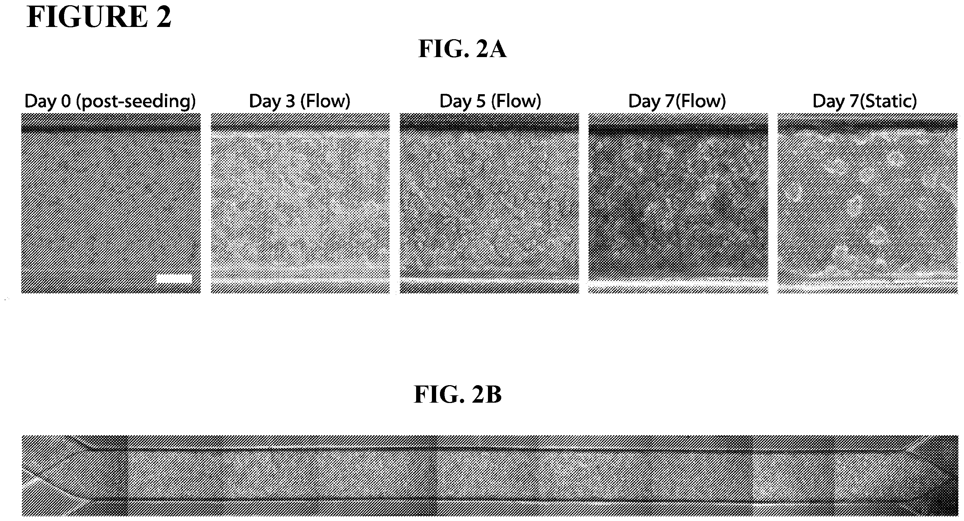

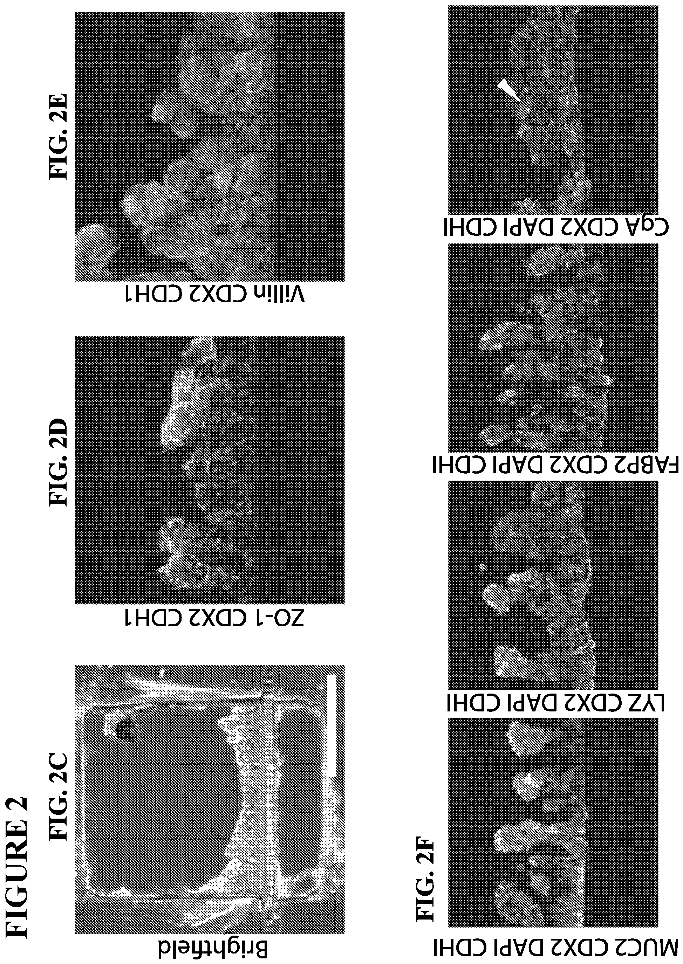

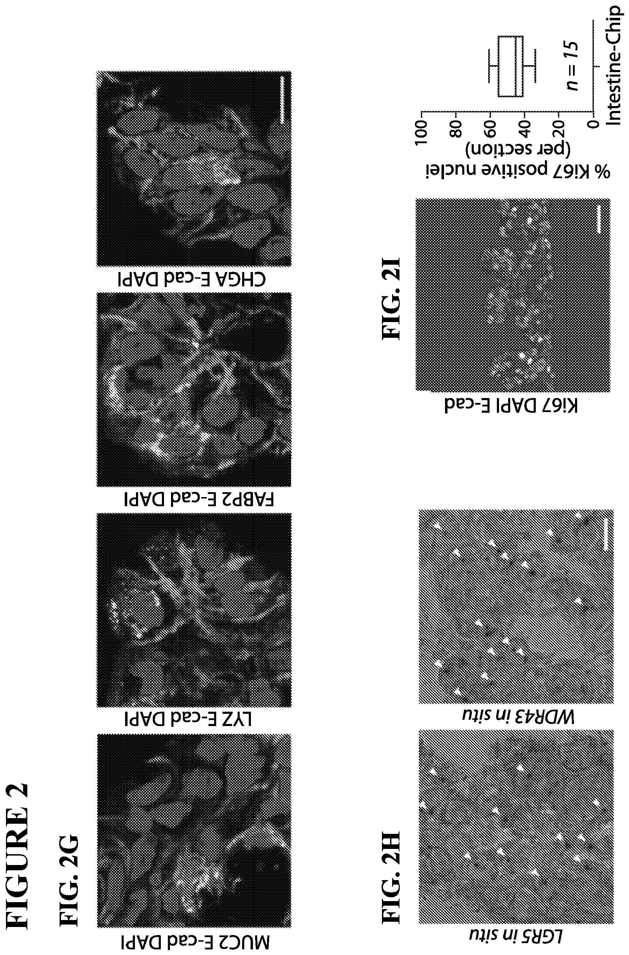

**Figure 2****.** a) Representative phase contrast image of HIO-derived epithelial cells seeded into the Chip immediately after seeding. Cells were then exposed to continual media flow at 30µl/hr and imaged after 3, 5 and 7 days. Static Chip was imaged after 7 days. b) Stitched phase contrast image of HIO-epithelial cells that were exposed to continual media flow at 30µl/hr for 5 days. c) Representative brightfield image of cross section of Chip that was exposed to continual media flow at 30 µl/hr for 14 days. d) Representative fluorescent image showing E-Cadherin (blue), CDX2 (red) and ZO1 (green) and e) E-Cadherin (blue), CDX2 (red), and villin (green) in cross section of Chip under conditions similar to c). f) Representative fluorescent images showing E-Cadherin (gray), CDX2 (red) and Muc2, lysozyme, FABP2 and chromogranin A (all green) in cross section of Chip under conditions similar to c). g) Representative fluorescent images showing E-cadherin/CDH1 (red), and MUC2, lysozyme, FABP2, and chromogranin A (all green), counterstained with DAPI (blue), in cross-section of Chips that were exposed to continual media flow at 30 mL/h for 7 days. Scale bar ¼ 10 mm. h) Representative images of in situ hybridization for LGR5Þ and WDR43Þ (white arrows) in conditions similar to F. Scale bar ¼ 10 mm. i) Representative fluorescent image showing E-cadherin/CDH1 (blue), Ki67 (green), and DAPI (blue) in conditions similar to F. Scale bar ¼ 100 mm. Graph shows percent Ki67Þ nuclei per section.

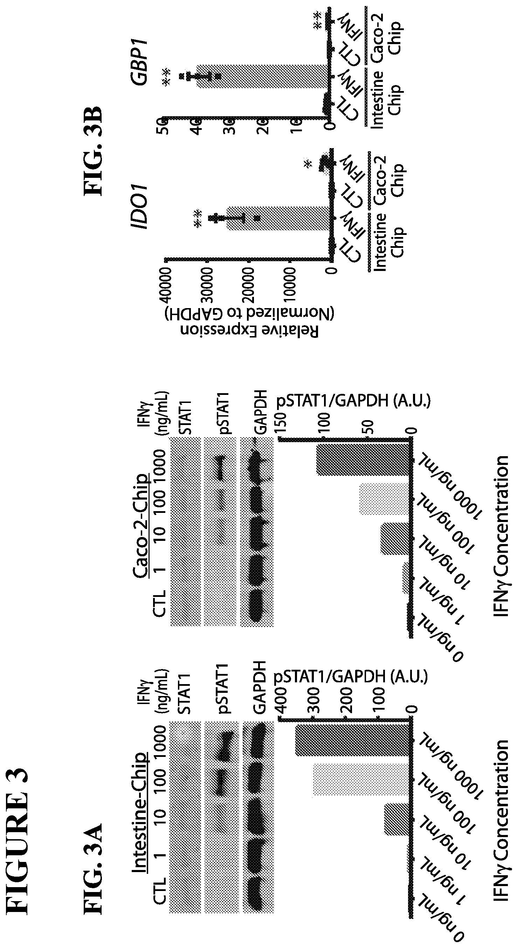

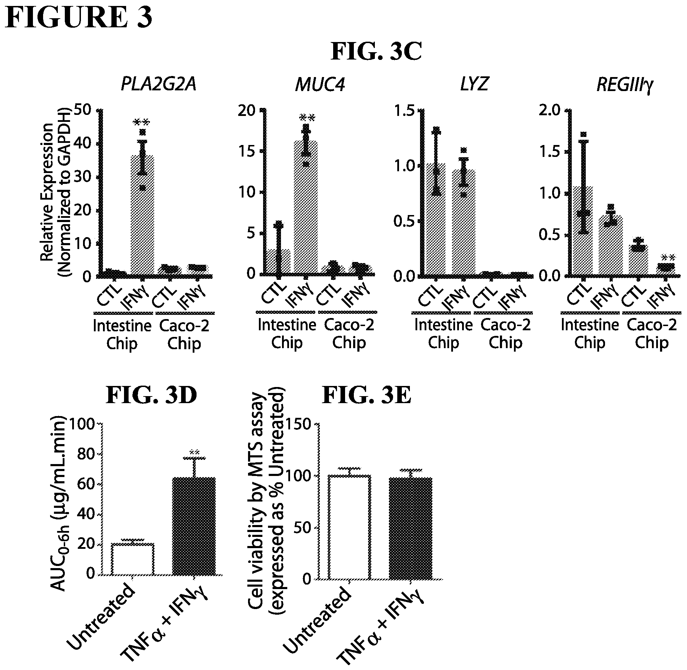

**Figure 3****.** a) Western blot analysis showing phosphorylation of STAT1 in Chips containing either HIO-derived intestinal epithelial or Caco2 cells in response to exposure of increasing concentrations (0-1000ng/ml) of IFNγ in the lower channel for 1 hour. Both GAPDH and STAT1 were used as loading controls. Image J analysis was used to quantify levels of phosphorylation of STAT1 compared to GAPDH b) Quantitative reverse transcription-polymerase chain reaction analyses of *IDO1* and *GBP1* and c) *PLA2G2a, Muc4, lysozyme, Reg3γ*in Chips incorporating either HIO-derived epithelial or Caco2 cells in response to exposure of 10ng/ml of IFNγ in the lower channel for 3 days. Three independent experiments were carried out; data represent mean and s.e.m; **P* < 0.05, ***P* < 0.01 compared to respective untreated controls. (D) Area under the curve of fluorescein isothiocyanate-dextran 4 kDa permeation over 6 hours was statistically higher (P < .01) in Intestine-Chip treated with IFN-g and TNF-a compared with untreated. (E) Following the permeability studies, MTS cytotoxicity assay was carried out and showed no statistical difference between Intestine-Chips treated with IFN-g and TNF-a or untreated. Two independent experiments were carried out; data represent mean ± SEM. **P < .01. AUC, area under the curve; CTL, control; GAPDH, glyceraldehyde-3-phosphate dehydrogenase.
**Figure 4****.** a) Phase contrast image and b) fluorescent image showing E-cadherin (green), CDX2 (red) counterstained with DAPI (blue) of intact HIO that was incorporated into the Chip and imaged 3 days later. c) Phase contrast image showing both epithelial and mesenchymal cells that arose from intact HIOs that were incorporated the Chip and imaged 4 days later. Yellow dotted lines represent mesenchymal cells in Chip.
**Figure 5****.** a) Representative phase contrast image of HIO-derived epithelial cells seeded into the chip immediately after seeding. Cells were then exposed to continual media flow of 60 µl/hr and imaged after 3, 5 and 7 days.
**Figure 6****.** a) Representative fluorescent images showing E-Cadherin (gray), CDX2 (red) and Muc2, lysozyme, FABP2 and chromogranin A (all green) in cross section of Chips incorporating Caco2 cells that were exposed to continual media flow of 30 µl/hr and imaged after 8 days.
**Figure 7****.** Colon on a Chip. In these experiments, 269 P34 HCO 5.63 × 10^6 cells were sorted, with seeding of 3.15 × 10^5 cells per chip. A dhered for overnight and regulated with media+SB202190 and A83-01 + iRock.
**Figure 8****.** Intestinal cells in transwell system. Cells can be seeded into transwell system. Here, 4 different lines were seeded on transwells and TEER was measured for 12 days. Each line represents an individual transwell and there were 8 transwell measured in each group.
**Figure 9****.** Growth of intestinal cells. Represntative mages of 83i cells when they grow over the various timepoints.
**Figure 10****.** Cryopreserved cells are viable upon thawing and survive when seeded onto transwells.
**Figure 11****.** Generation of iPSC cell line from Inflammatory Bowel Disease subjects including early onset and very early onset subjects.

### DETAILED DESCRIPTION OF THE INVENTION

Unless defined otherwise, technical and scientific terms used herein have the same meaning as commonly understood by one of ordinary skill in the art to which this invention belongs. Singleton et al., Dictionary of Microbiology and Molecular Biology 3rd ed., Revised, J. Wiley & Sons (New York, NY 2006); and Sambrook and Russel, Molecular Cloning: A Laboratory Manual 4th ed., Cold Spring Harbor Laboratory Press (Cold Spring Harbor, NY 2012), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described.

As described, Inflammatory Bowel Diseases (IBDs) are complex, multifactorial disorders characterized by chronic relapsing intestinal inflammation. The two major subtypes of IBD are Ulcerative Colitis (UC) and Crohn's Disease (CD). Ulcerative Colitis and CD are important worldwide health problems, with an incidence in Europe of 12.7 and 24.3 per 100,000 person-years, respectively, and prevalence of 0.5 and 1.0%. Although the exact etiology is still not completely known, recent studies have indicated that personal genetic susceptibility, environment, intestinal microbiota, and immune system are all involved in the pathogenesis of IBDs.

Approximately 20-25% of patients with IBD are diagnosed before 16 years old. The onset of intestinal inflammation in children can affect development and growth. Age of onset can also provide information on type of IBD and associated genetic features. Although most cases of IBD are correlated with a polygenic contribution toward genetic susceptibility, there is a spectrum of rare genetic disorders that produce IBD-like intestinal inflammation. Early-onset inflammatory bowel diseases (EO-IBD) (e.g., before 5 years) and very-early-onset inflammatory bowel diseases (VEO-IBD) (e.g., before 2 years) are rare, particularly severe disease presentations. There are few case series that report early-onset of IBDs, starting within the first year of life with typical characteristics of CD, instead they are often described as CD or CD-like. The majority of VEO-IBD are caused by genetic defects. As monogenic diseases, they are good candidates for studying IBD etiology. Nevertheless, the genetic variants that cause these disorders have a wide effect on gene function and are so rare in allele frequency that the genetic signals are not detected in GWAS of patients with IBD. Monogenic defects have been found to alter intestinal immune homeostasis via several mechanisms. These include disruption of the epithelial barrier and the epithelial response, as well as reduced clearance of bacteria by neutrophil granulocytes and other phagocytes. Other single-gene defects induce hyperinflammation or autoinflammation or disrupted T- and B-cell selection and activation. Hyperactivation of the immune response can result from defects in immune inhibitory mechanisms, such as defects in IL-10 signaling or dysfunctional regulatory T-cell activity.

Modeling IBD in cellular and cellular organoid systems presents challenges considering the physiological structure that is the environmental context for the disease. The human gastrointestinal (GI) tract comprises the foregut, midgut, and hindgut. Each region gives rise to different tissues and organs. The foregut endoderm gives rise to the epithelium of the oral cavity, pharynx, esophagus, stomach, liver, pancreas, and proximal duodenum. The midgut and hindgut endoderm give rise to the epithelium of the distal duodenum, jejunum, ileum, colon, rectum, and anal canal, as well as the epithelial lining of the bladder and urethra. In vitro organoid cultures are generated from stem or progenitor cells. Such organoids have capabilities for long-term growth, cellular diversity, function, and spatial organization specific to the organ they represent, and they have been used to study endoderm-derived GI organs. Organoid technologies now enable the growth of diverse primary human tissues in vitro. For example, long-term organoid cultures have been used to examine the stem cell niche, cellular differentiation, interactions between cells, and physiological functions. They have also been used to model malignancy, infection, and inflammation, as well as in toxicology studies. Despite this remarkable repertoire of capabilities, cellular and cellular organoid systems can be limited by historical reliance on anatomical landmarks and the lack of more precise molecular markers to distinguish foregut, midgut, and hindgut. Classification of foregut, midgut, and hindgut has been based on the development of the anterior and posterior intestinal portals and the source of mesenteric blood supply. As such, this classification has made it difficult to develop methods to generate these cells and tissues in vitro from PSCs. Human embryonic and induced PSC (ESC and iPSC)-derived organoids are an ideal platform for more precisely defining cellular fates in the intestine. ESCs or iPSCs are differentiated into intestine or gastric tissue using stepwise differentiation that mimick stages of embryonic development. Similar to culture of tissue-derived epithelial and epithelial-mesenchymal organoids, application of a 3D extracellular matrix with media that supported high levels of Wnt signaling has been applied.

Advancing these studies is the combination of intestinal organoid technology with microfluidic technology. For example, small microfluidic chips designed with two parallel microchannels that are separated by a porous membrane to allow for the continuous flow of media recreates some components of the *in vivo* microenvironment. The engineering also allows the tuning and control of the microenvironment to direct cell fate. Given that IPSCs can be generated from any individual, iPSC-derived organoids were incorporated into these systems to study intestinal cells from virtually any individual or patient, including early onset and very early onset (monogenic) IBD subjects. Herein, the Inventors demonstrate that fate specification towards human intestinal organoids (HIOs) or human colonic organoids (HCOs) can be guided by alteration of Wnt signaling, wherein BMP inhibition abrogates the ability of WNT and FGF to promote a posterior endoderm fate. This allows for the generation of other patient specific cell types such as macrophages, dendritic cells, neutrophils that could also be incorporated into the Chip and used to study multicellular interactions. Furthermore, the Inventors can reliably reprogram from both blood samples and lymphoblastoid cell lines (LCLs) to form IPSCs. This provides robust opportunities to collect samples from rare disease subjects, including early onset and very early onset IBD patients. Whether from individual subjects via blood draw, or LCLs characterized in repositories linked to patient clinical history and long-term genotype-phenotype data, the aforementioned platforms permit studies into patient groups otherwise hard to capture.

Described herein is a method including generation of HIOs or HCOs from iPSCs, including differentiation of iPSCs into definitive endoderm, epithelial structures and organoids. In various embodiments, induction of definitive endoderm includes culturing of iPSCs with Activin A and Wnt3A, for 1, 2, 3, 4 or more days, and increasing concentrations of FBS over time. In various embodiments, induction of definitive endoderm includes culturing of iPSCs with Activin A (e.g., 100ng/ml), Wnt3A (25ng/ml), for 1, 2, 3, 4 or more days, and increasing concentrations of FBS over time (0%, 0.2% and 2% on days 1, 2 and 3 respectively). For example, induction of definitive endoderm includes culturing of iPSCs with Activin A (e.g., 100ng/ml), Wnt3A (25ng/ml), for 1, 2, 3, 4 or more days, and increasing concentrations of FBS over time (0%, 0.2% and 2% on days 1, 2 and 3 respectively). In various embodiments, the concentration of Activin A includes about 0-25 ng/ml, about 25-50 ng/ml, about 50-75 ng/ml, about 100-125ng/ml, about 125-150 ng/ml. In various embodiments, the concentration of Wnt3A includes about -25 ng/ml, about 25-50 ng/ml, about 50-75 ng/ml, about 100-125ng/ml, about 125-150 ng/ml. In various embodiments, the concentrations of FBS over time include about 0%-0.2%, about 0.2%-0.5%, about 0.5%-1%, about 1%-2%, and 2% or more on each of days 1, 2 and 3 respectively. In various embodiments, formation of hindgut includes culturing of definitive endoderm cells for 1, 2, 3, 4 or more days in media such as Advanced DMEM/F12 with FBS and FGF4. In various embodiments, formation of hindgut includes culturing of definitive endoderm cells for 1, 2, 3, 4 or more days in media include FBS at a concentration of 0%-0.2%, about 0.2%-0.5%, about 0.5%-1%, about 1%-2%, and 2% or more and concentration of FGF4 at about 50-100 ng/ml, about 100-250 ng/ml, about 250-500ng/ml, and 500 ng/ml or more. For example, formation of hindgut can include culturing of definitive endoderm cells for 1, 2, 3, 4 or more days in media such as Advanced DMEM/F12 with 2% FBS and FGF4 (500ng/ml). In various embodiments, Wnt3A, CHIR99021 or both are added. In various embodiments, the concentration of Wnt3A includes about 100-250 ng/ml, about 250-500ng/ml, and 500 ng/ml, the concentration of CHIR99021 is about 0.5-1 µM, about 1-1.5 µM, about 1.5-2 µM or 2 µM or more are added. For example, both Wnt3A (500ng/ml), CHIR99021 (2 µM) or both are added.

In various embodiments, after about 3-4 days, the method includes isolation of organoids including free floating epithelial spheres and loosely attached epithelial tubes.

In various embodiments, the isolated organoids are suspended in Matrigel and then overlaid in intestinal medium containing CHIR99021, noggin, EGF and B27. In various embodiments, the isolated organoids are suspended in Matrigel and then overlaid in intestinal medium containing CHIR99021, noggin, EGF and B27 to generate human intestinal organoids (HIOs). In various embodiments, the concentration of CHIR99021 is about 0.5-1 µM, about 1-1.5 µM , about 1.5-2 µM or 2 µM, the concentration of noggin at about 50-100 ng/ml, about 100-250 ng/ml is about 250-500ng/ml, and 500 ng/ml or more, the concentration of EGF at about 50-100 ng/ml, about 100-250 ng/ml, about 250-500ng/ml, and 500 ng/ml or more and the concentration of B27 is about 0.25X-0.5x, about 0.5-1X, about 1X-2X or 2X or more. For example, the media contains CHIR99021 (2 µM), noggin (100ng/ml) and EGF (100ng/ml) and B27 (1X). In various embodiments, HIOs are passaged every 7-10 days thereafter.

In various embodiments, after about 3-4 days, the method includes isolation of organoids including free floating epithelial spheres and loosely attached epithelial tubes. In various embodiments, the isolated organoids are suspended in Matrigel and then overlaid in intestinal medium containing EGF and BMP to generate human colonic organoids (HCOs). In various embodiments, EGF and BMP protein are added for 1-4 days, including for example 3 days. In various embodiments, the concentration of EGF is about 50-100 ng/ml, about 100-250 ng/ml, about 250-500ng/ml, and 500 ng/ml or more, including for example, an EGF concentration of about 100ng/ml. In various embodiments, the concentration of BMP is about 50-100 ng/ml, about 100-250 ng/ml, about 250-500ng/ml, and 500 ng/ml or more, including for example, an EGF concentration of about 100ng/ml.

In various embodiments, the population of intestinal cells are an organized population including features of intestinal organs. In various embodiments, the inestitinal cells are organized into villi. In various embodiments, the villi are lined by all four epithelial cell lineages of the small intestine (absorptive, goblet, enteroendocrine, and Paneth). In various embodiments, the population of intestinal cells express one or more of PDX1, GATA4, DEFA5. In various embodiments, the population of intestinal cells possess barrier function, drug-metabolizing cytochrome P450 activity, and/or apical mucus secretion. In various embodiments, the population of colonic cells are an organized population including features of the colon. In various embodiments, the colonic cells are absorptive cells, goblet cells, vacuolated cells, M cells, undifferentiated crypt cells, vaeoliated cells, deep crypt secretory cells, enteroendocrine cells. In various embodiments, the population of colonic cells express one or more of SATB2, MUC5B, MUC2, INSL5, HOXA13, CKB, FXYD3, and HOXB 13.

In various embodiments, the iPSCs are derived from cells obtained from a subject afflicted with early onset or very early onset Inflammatory Bowel Disease (IBD). In various embodiments, the iPSCs are derived from cells obtained from a subject with a genetic predisposition to early onset or very early onset Inflammatory Bowel Disease (IBD). This includes, for example, mutations in CCL14, MMP28, LRCH3, SIGLEC12, ZNF425, and others genetic loci known to one of skill in the art to be related to early onset or very early onset IBD. In various embodiments, early onset IBD includes a subject less than 18, 17, 16, 15, 14, or 13 or less years of age. In various embodiments, very early onset IBD include a subject less than 13, 12, 11, 10, 9, 8, 7, 6, 5, 4, 3, 2 or less years of age. In various embodiments, the iPSCs are derived from cells obtained from a subject afflicted with colon cancer. In various embodiments, the iPSCs are derived from cells obtained from a subject with genetic predisposition to colon cancer.

In various embodiments, the iPSCs obtained from a subject include cells reprogrammed from a blood draw. In various embodiments, cells reprogrammed from a blood draw are made by a method including contacting a quantity of blood cells with one or more vectors encoding a reprogramming factor, and delivering a quantity of reprogramming factors into the blood cells, culturing the blood cells in a reprogramming media, and further wherein delivering the reprogramming factors, and culturing in a reprogramming media generates blood cell derived induced pluripotent stem cells (iPSCs). Further information on iPSC reprogramming is found in U.S. App. No. 15/184,241 and PCT App. No. PCT/US2017/038041. In various embodiments, iPSCs obtained from a subject include cells reprogrammed lymphoblastoid cells or lymphoblast cell lines (LCLs). Further information on iPSC reprogramming is found in Barrett, R. et al. Reliable Generation of Induced Pluripotent Stem Cells from Human Lymphoblastoid Cell Lines. Stem Cells Transl Med. 2014 Dec;3(12): 1429-34.

In various embodiments, generating iPSCs includes providing a quantity of cells, delivering a quantity of reprogramming factors into the cells, culturing the cells in a reprogramming media for at least 4 days, wherein delivering the reprogramming factors, and culturing generates induced pluripotent stem cells. In certain embodiments, the cells are primary culture cells. In other embodiments, the cells are blood cells (BCs). In certain embodiments, the blood cells are T-cells. In other embodiments, the blood cells are non-T-cells. In other embodiments, the cells are mononuclear cells (MNCs), including for example peripheral blood mononuclear cells (PBMCs). In other embodiments, the cells are primary granulocytes, monocytes and B-lymphocytes.

In certain embodiments, the reprogramming factors are Oct-4, Sox-2, Klf-4, c-Myc, Lin-28, SV40 Large T Antigen ("SV40LT"), and short hairpin RNAs targeting p53 ("shRNA-p53"). In other embodiments, these reprogramming factors are encoded in a combination of vectors including pEP4 E02S ET2K, pCXLE-hOCT3/4-shp53-F, pCXLE-hSK, pCXLE-hUL and pCXWB-EBNA1. This includes, for example, using about 0.5 - 1.0 ug pCXLE-hOCT3/4-shp53, 0.5 - 1.0 ug pCXLE-hSK, 0.5 - 1.0 ug pCXLE-UL, about 0.25 - 0.75 ug pCXWB-EBNA1 and 0.5 - 1.0 ug pEP4 E02S ET2K. This includes, for example, using 0.83ug pCXLE-hOCT3/4-shp53, 0.83ug pCXLE-hSK, 0.83ug pCXLE-UL, 0.5ug pCXWB-EBNA1 and 0.83 ug pEP4 E02S ET2K, wherein the stoichiometric ratio of SV40LT (encoded in pEP4 E02S ET2K) and EBNA-1 (encoded in pCXWB-EBNA1) supports the reprogramming of non-T cell component of blood, including peripheral blood mononuclear cells. In various embodiments, the reprogramming media is embryonic stem cell (ESC) media. In various embodiments, the reprogramming media includes bFGF. In various embodiments, the reprogramming media is E7 media. In various embodiments, the reprogramming E7 media includes L-Ascorbic Acid, Transferrin, Sodium Bicarbonate, Insulin, Sodium Selenite and/or bFGF. In different embodiments, the reprogramming media comprises at least one small chemical induction molecule. In certain other embodiments, the reprogramming media includes PD0325901, CHIR99021, HA-100, and A-83-01. In other embodiments, the culturing the blood cells in a reprogramming media is for 4-30 days.

In various embodiments, the iPSCs are capable of serial passaging as a cell line. In various embodiments, the iPSCs possess genomic stability. Genomic stability can be ascertained by various techniques known in the art. For example, G-band karyotyping can identify abnormal cells lacking genomic stability, wherein abnormal cells possess about 10% or more mosaicism, or one or more balanced translocations of greater than about 5, 6, 7, 8, 9, 10 or more Mb. Alternatively, genomic stability can be measured using comparative genomic hybridization (aCGH) microarray, comparing for example, iPSCs against iPSCs from a nonblood cell source such as fibroblasts. Genomic stability can include copy number variants (CNVs), duplications/deletions, and unbalanced translocations. In various embodiments, iPSCs exhibit no more than about 3, 4, 5, 6, 7, 8, 9, 10, 11, 12, 13, 15, 16, 17, 18, 19, or 20 Mb average size of amplification and deletion. In various embodiments, BC-iPSCs exhibit no more than about 20-30 Mb average size of amplification and deletion. In various embodiments, iPSCs exhibit no more than about 30-40 Mb average size of amplification and deletion. In various embodiments, iPSCs exhibit no more than about 40-50 Mb average size of amplification and deletion. In various embodiments, the average number of acquired de novo amplification and deletions in iPSCs is less than about 5, 4, 3, 2, or 1. For example, de novo amplification and deletions in fib-iPSCs are at least two-fold greater than in PBMC-iPSCs. In various embodiments, the methods produce iPSC cell lines collectively exhibiting about 20%, 15%, 10%, 5% or less abnormal karyotypes over 4-8, 9-13, 13-17, 17-21, 21-25, or 29 or more passages when serially passaged as a cell line.

In other embodiments, the reprogramming factors are delivered by techniques known in the art, such as nuclefection, transfection, transduction, electrofusion, electroporation, microinjection, cell fusion, among others. In other embodiments, the reprogramming factors are provided as RNA, linear DNA, peptides or proteins, or a cellular extract of a pluripotent stem cell. In certain embodiments, the cells are treated with sodium butyrate prior to delivery of the reprogramming factors. In other embodiments, the cells are incubated or 1, 2, 3, 4, or more days on a tissue culture surface before further culturing. This can include, for example, incubation on a Matrigel coated tissue culture surface. In other embodiments, the reprogramming conditions include application of norm-oxygen conditions, such as 5% O₂, which is less than atmospheric 21% O₂.

In various embodiments, the reprogramming media is embryonic stem cell (ESC) media. In various embodiments, the reprogramming media includes bFGF. In various embodiments, the reprogramming media is E7 media. In various embodiments, the reprogramming E7 media includes L-Ascorbic Acid, Transferrin, Sodium Bicarbonate, Insulin, Sodium Selenite and/or bFGF. In different embodiments, the reprogramming media comprises at least one small chemical induction molecule. In different embodiments, the at least one small chemical induction molecule comprises PD0325901, CHIR99021, HA-100, A-83-01, valproic acid (VPA), SB431542, Y-27632 or thiazovivin ("Tzv"). In different embodiments, culturing the BCs in a reprogramming media is for at least 4, 5, 6, 7, 8, 9, 10, 11, 12, 13, 14, 15, 16, 17, 18, 19, 20, 21, 22, 23, 24, 25, 26, 27, 28, 29, or 30 days.

Described herein is a population of intestinal cells. In various embodiments, the population includes an organized structure. In various embodiments, the organized structure includes villi. In various embodiments, the villi are lined by one or more epithelial cell lineages selected from the group consisting of: absorptive, goblet, enteroendocrine, and Paneth cells. In various embodiments, the population of intestinal cells express one or more of PDX1, GATA4, DEFA5. In various embodiments, the organized structure possesses barrier function, cytochrome P450 activity, and/or apical mucus secretion. In various embodiments, the intestinal cells human intestinal organoids (HIOs) or cells derived from HIOs that are disaggregated into single cells and purified based on CD326+ expression. In various embodiments, the HIOs are derived from iPSCs by a method including generation of definitive endoderm by culturing iPSCs in the presence of Activin A and Wnt3A, differentiation into hindgut by culturing definitive endoderm in the presence of FGF and either Wnt3A or CHIR99021, collection of epithelial spheres or epithelial tubes, suspension of epithelial spheres or epithelial tubes in Matrigel, and culturing in the presence of CHIR99021, noggin and EGF. In various embodiments, the iPSCs are derived from cells obtained from a subject afflicted with early onset or very early onset Inflammatory Bowel Disease (IBD). In various embodiments, the iPSCs are derived from cells obtained from a subject with a genetic predisposition to early onset or very early onset Inflammatory Bowel Disease (IBD). In various embodiments, early onset IBD includes a subject less than 18, 17, 16, 15, 14, or 13 or less years of age. In various embodiments, very early onset IBD include a subject less than 13, 12, 11, 10, 9, 8, 7, 6, 5, 4, 3, 2 or less years of age. In various embodiments, the population of intestinal cells are an organized population including features of intestinal organs. In various embodiments, the inestitinal cells are organized into villi. In various embodiments, the villi are lined by all four epithelial cell lineages of the small intestine (absorptive, goblet, enteroendocrine, and Paneth). In various embodiments, the population of intestinal cells express one or more of PDX1, GATA4, DEFA5. Further described herein is a cryopreserved solution including the population of intestinal cells.

Described herein is a population of colonic cells. In various embodiments, the population includes an organized structure. In various embodiments, the population of colonic cells are an organized population including features of the colon. In various embodiments, the colonic cells are absorptive cells, goblet cells, vacuolated cells, M cells, undifferentiated crypt cells, vaeoliated cells, deep crypt secretory cells, enteroendocrine cells. In various embodiments, the population of colonic cells express one or more of SATB2, MUC5B, MUC2, INSL5, HOXA13, CKB, FXYD3, and HOXB13. In various embodiments, the intestinal cells are human colonic organoids (HCOs) or cells derived from HCOs that are disaggregated into single cells. In various embodiments, the HCOs are derived from iPSCs by a method including generation of definitive endoderm by culturing iPSCs in the presence of Activin A and Wnt3A, differentiation into hindgut by culturing definitive endoderm in the presence of FGF and either Wnt3A or CHIR99021, collection of epithelial spheres or epithelial tubes, suspension of epithelial spheres or epithelial tubes in Matrigel, and culturing in the presence of EGF and BMP. In various embodiments, the iPSCs are derived from cells obtained from a subject afflicted with early onset or very early onset Inflammatory Bowel Disease (IBD). In various embodiments, the iPSCs are derived from cells obtained from a subject with a genetic predisposition to early onset or very early onset Inflammatory Bowel Disease (IBD). In various embodiments, early onset IBD includes a subject less than 18, 17, 16, 15, 14, or 13 or less years of age. In various embodiments, very early onset IBD include a subject less than 13, 12, 11, 10, 9, 8, 7, 6, 5, 4, 3, 2 or less years of age. In various embodiments, the iPSCs are derived from cells obtained from a subject afflicted with colon cancer. In various embodiments, the population of colonic cells express one or more of SATB2, MUC5B, MUC2, INSL5, HOXA13, CKB, FXYD3, and HOXB13. Further described herein is a cryopreserved solution including the population of colonic cells.

Described herein is a method of compound screening, including contacting a quantity of intestinal or colon cells with one or more test compounds, measuring one or more parameters, and selecting one or more test compounds based on the measured one or more parameters. In various embodiments, the intestinal or colon cells are differentiated from blood cell-derived induced pluripotent stem cells (iPSCs). In various embodiments, the one or more parameters include barrier function, drug-metabolizing cytochrome P450 activity, and/or apical mucus secretion.

Further information can be found in PCT App. No. PCT/US2017/016098, PCT/US2017/016079, and PCT/US2017/038041.

### Example 1

### Cell lines and culturing conditions

Two IPSC lines, the CS83iCTR-33n1 and CS688iCTR-n5 line were obtained from the IPSC Core in Cedars Sinai. Both lines were fully characterized and were karyotypically normal. All IPSC lines were maintained in an undifferentiated state on Matrigel coated plates in mTeSR1 media (Stem Cell Technologies) under feeder free conditions. All IPSC cultures were tested monthly for mycoplasma contamination.

### Example 2

### Small microengineering Chip microfabrication

The Inventors fabricated the Chip by using modified methods for Chip microfabrication as previously described. Briefly, PDMS pre-polymer was mixed at a 10:1 ratio of PDMS base to curing agent, wt/wt using a planetary mixer (Thinky ARE-310). PDMS pre-polymer was then cast onto molds forming the microchannels of the upper layer (1,000 um wide × 1,000um high) and lower layer (1,000um wide × 200um high). The membrane was cast onto a silicon mold that was fabricated using photolithography and deep reactive ion etching, resulting in 7um pores. The components were cured overnight and removed from the mold. The upper layer, membrane, and lower layer were permanently bonded via plasma bonding to form the complete Chip.

### Example 3

### Generation of human intestinal organoids from induced pluripotent stem cells

The generation of human intestinal organoids (HIOs) from IPSCs involves a multistep technique whereby IPSCs with directed to form definitive endoderm, epithelial structures and ultimately organoids. To induce definitive endoderm formation, all iPSCs were cultured with a high dose of Activin A (100ng/ml, R&D Systems) with increasing concentrations of FBS overtime (0%, 0.2% and 2% on days 1, 2 and 3 respectively). Wnt3A (25ng/ml, R&D Systems) was also added on the first day of endoderm differentiation. To induce hindgut formation, cells were cultured in Advanced DMEM/F12 with 2% FBS and FGF4 (500ng/ml, R&D Systems) and with either Wnt3A (500ng/ml, R&D Systems) or CHIR99021 (3 µM, Tocris). After 3-4 days, free floating epithelial spheres and loosely attached epithelial tubes became visible and were harvested. These epithelial structures were subsequently suspended in Matrigel and then overlaid in intestinal medium containing CHIR99021 (2□M, Tocris), noggin and EGF (both 100ng/ml, all R&D Systems) and B27 (1X, Invitrogen). HIOs were passaged every 7-10 days thereafter.

### Example 4

### Seeding and maintenance of Intestine-Chip

To seed intestinal epithelial cells into the Chip, HIOs were first dissociated and the intestinal epithelial cells were then obtained using fluorescent activated cell sorting. 24 hours prior to sorting, ROCK inhibitor (10µM, Tocris) was added to HIO culture media. The following day, HIOs were removed from Matrigel and subsequently incubated in TrypLE Express (Life Technologies) for between 30 min until the organoids are completely disassociated to a single cell suspension. These cells were then passed through a 30mm filter and stained with CD326 (Biolegend) for 30min. Cells were then positively sorted for CD326. Cells were collected and resuspened to a density of 5×10⁶/ml in intestinal media containing ROCK inhibitor (10mM, Tocris), SB202190 (10mM, Tocris) and A83-01 (500nM, Tocris). Dead/non-adhered cells were removed after 3-6 hours by flushing media through the device and flow was started 3-6 hrs later at a rate 30ul/hr. ROCK inhibitor was removed from media 24 hours post seeding.

### Example 5

### Quantitative real time-PCR

PBS was flushed through both the upper and lower channels and total RNA from the Intestine-Chip was extracted *in situ* with the RNeasy mini kit (Qiagen, USA). cDNA was generated from 1 µg of RNA using the Omniscript RT Kit (Qiagen). qRT-PCR was performed using SYBR^{®} Select Master Mix (Applied Biosystems) on a BioRad CFX384 Real-Time System. Primer sequences as follows:
IDO1 F-ACACTTTGCTAAAGGCGCTG [SEQ ID NO: 1]
IDO R-TGCCTTTCCAG CCAGACAAA [SEQ ID NO: 2]
GBP1 F-AAACTTCAGGAACAGGAGCAAC [SEQ ID NO: 3]
GBP1 R-GGTACATGCCTTTCGTC GTCT [SEQ ID NO: 4]
PLA2G2A F-CAAGTTTAGCAACTCGGGGA [SEQ ID NO: 5]
PLA2G2A R-TCTAGCAAAACAGGTGGCAG [SEQ ID NO: 6]
Muc4 F-AGACGCACAGCCACATCAC [SEQ ID NO: 7]
Muc4 R-GGGAAACTCCTCTCTCAGGC [SEQ ID NO: 8]
Lysozyme F-TTTCTGTTACGGTCCAGGGC [SEQ ID NO: 9]
Lysozyme R-ACACATCCAGTTTGCTAGGCT [SEQ ID NO: 10]
Reg3 F- CCTTTAGTGACCCGATTGCCT [SEQ ID NO: 11]
Reg3 R-TCTTCACCTTGAACCTGACACAG [SEQ ID NO: 12].

### Example 6

### Immunohistochemistry and microscopy.

HIOs were fixed in 4% paraformaldehyde (Electron Microscopy Sciences, USA), transferred to 30% sucrose, embedded in HistoPrep (Thermo Fisher Scientific) and cut into 20□m sections. Sections were blocked in 10% donkey serum (Jackson ImmunoResearch) with 0.5% Triton X-100 and incubated with primary antibodies either for either 3hrs at room temperature or overnight at 4°C. Sections were then rinsed and incubated in species-specific AF488, AF594 or AF647-conjugated secondary antibodies followed by Hoechst 33258 (0.5 µg/ml; Sigma) to counterstain nuclei, and were imaged using a Leica DM6000 B microscope. Intestine-Chips were flushed through both the upper and lower channels, and cells were fixed with 4% paraformaldehyde for 20 min without flow. Intestine-Chips cultured under static conditions were immunostained and imaged in a similar manner as above. To obtain cross-section images, a vibrotome was used to obtain 100□m sections of the Intestine-Chip. These sections were blocked in 10% donkey serum with 0.5% Triton X-100 and incubated with primary antibodies for 48 hours at 4°C. Sections were rinsed, incubated in species specific AF488, AF594 and AF647 followed by Hoechst 33258, and were imaged using a Nikon A1R Eclipse Ti Confocal Microscope.

### Example 7

### Western blotting

PBS was flushed through both the upper and lower channels and total protein was extracted *in situ* using cell lysis buffer (Cell Signaling cat# 9803s) supplemented with protease and phosphates inhibitor (Sigma, # MSSAFE). Protein concentration was then determined and equal amounts of protein lysates were heat denatured and separated on a 10% mini-protean precast gel (Bio-Rad, #456-8034). Gel was then transferred on to midi format 0.2µM Nitrocellulose Membrane (Bio-Rad cat#170-4157). Blot was blocked in Odyssey Blocking Buffer (LI-COR Cat # 92750000) for 1 hr and then incubated in primary antibody for STAT1, pSTAT1 and GAPDH overnight at 4°C. After incubation with infrared conjugated secondary antibodies (LI-COR #925-32211, LI-COR #925-68070 and LI-COR #926-32214) for one hour, blots were then washed with TBST and bands were visualized by scanning membrane on Odyssey Infrared Imaging System (Li-Cor Biosciences).

### Example 8

### Statistics analyses

All data are represented as mean ± s.e.m. An unpaired student's t test was completed using Excel, and control and IFNg treated Chips were compared to each other in each condition. Differences between groups were considered statistically significant when P < 0.05. No statistical method was used to predetermine sample size. The investigators were not blinded to allocation during experiments and outcome assessment. No randomization was made.

### Example 9

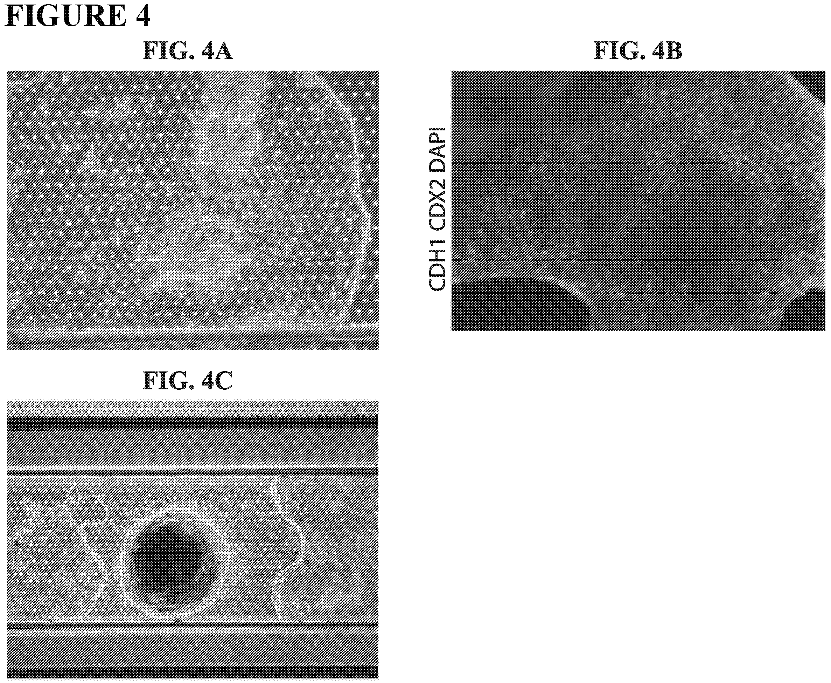

To develop the Intestine-Chip, the Inventors established a multi-step technique whereby IPSCs were differentiated into HIOs, disassociated to single cells, and subsequently incorporated into small microengineered Chips (Fig. 1a). IPSCs were induced to form three dimensional HIOs by culturing them with Activin A to induce definitive endoderm formation, Chir99021 and FGF4 to induce hindgut formation and ultimately EGF, noggin and Chir99021 to induce and maintain organoid formation. This protocol resulted in the robust generation of intestinal organoids which were polarized towards the lumen, were CDX2+/E-Cadherin+ immunopositive and in contrast to biopsy-derived organoids, contained mesenchymal cells as previously reported (Fig. 1b)⁵. The Chips used in this study were made of poly(dimethylsiloxane) (PDMS) containing an upper channel with a height and width of 1 mm separated from a parallel lower channel (0.2 mm high × 1 mm wide) by a thin (50 µm), porous (7µm pores), PDMS coated on the upper side with Matrigel (Fig. 1c). Initially, the Inventors attempted to place intact HIOs into the top channel of the Chip. Although some HIOs adhered to the PDMS membrane and formed a partial monolayer of intestinal epithelial cells (Fig. 4a and b), the majority did not adhere and indeed some HIOs gave rise to partial monolayers of mesenchymal cells (Fig. 4c) that appeared to impede the expansion of the epithelial cells (data not shown). Given that the number of epithelial and mesenchymal cells within HIO cultures can vary enormously and the presence of mesenchymal cells appears to impede the expansion of epithelial cells, the Inventors sought to refine the Inventors' approach. HIOs were disassociated to a single cell suspension and epithelial cell adhesion molecule (EpCAM/CD326) was used to positively select for HIO-derived epithelial cells using fluorescent activated cellular sorting. Concentrations of positively selected cells ranging from 2.5 - 7.5 ×10⁶ cells/ml were added to the Matrigel-coated top channel of each Chip and it was found that 6.25×10⁶ cells/ml gave a near continuous monolayer after 24 hrs (Fig. 1d). After the Inventors three days the Inventors observed of a fully confluent monolayer of intestinal epithelial cells along the entire channel of the Chip (Fig. 1e) and thus this concentration of cells was added in all future experiments (Fig. 1e).

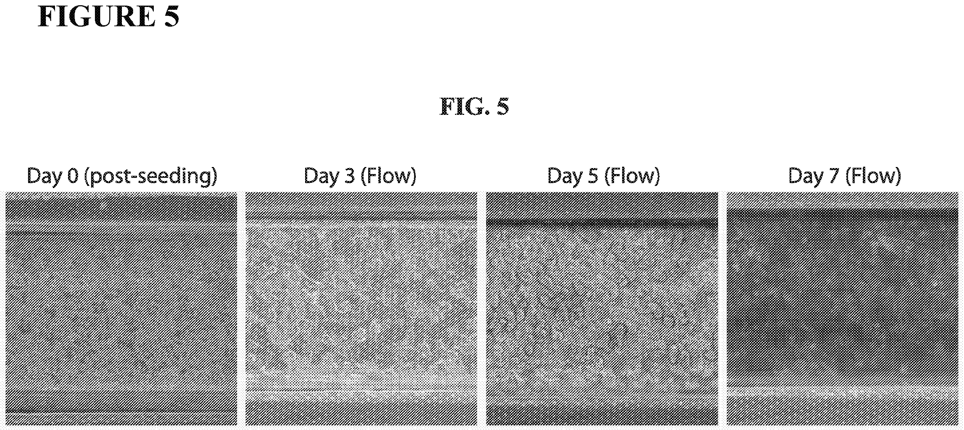

After establishing the conditions to successfully incorporate HIO-derived epithelial cells into the Intestine-Chip, the Inventors then wished to ascertain the optimal rate that media should continuously be flowed through the device. This continuous media flow mimics some of the *in vivo* microenvironment conditions and not only permits prolonged exposure to microbes, but enhances the differentiation of cells. Indeed it was previously shown that continuous flow of media caused Caco2 cells to spontaneously grow into folds that recapitulated intestinal villous -like structures. The Inventors used a similar flow rate of 30µl/hr that was previously reported and found that after 72 hours, the HIO-derived epithelial cells exhibited a more crenulated appearance along the entire length of the Chip, which became more pronounced after 5 and 7 days. Under static conditions, some small projections were observed after 7 days although they primarily remained as a monolayer (Fig. 2a and b). An increased flow rate of 60µl/hr did not appear to accelerate the process and so a flow rate of 30µl/hr was used in all subsequent studies (Fig. 5a). After 14 days, a cross section of the Intestine-Chip revealed a substantially thickened layer that appeared to have organized into villous-like projections (Fig. 2b). The presence of CDX2, E-Cadherin and polarized ZO1 immunostaining towards the apical aspect demonstrates these are polarized intestinal epithelial cells and illustrates that the top channel is representative of the luminal aspect of the intestine (Fig. 2c). The presence of villin staining demonstrates brush border differentiation (Fig. 2d). Given that it was previously reported that HIOs contained the major epithelial subtypes, the Inventors confirmed the presence of Paneth (lysozyme+) cells, goblet (Muc2+) cells, enteroendocrine (chromogranin A+) cells and (FABP2+) enterocytes (Fig. 2d) in the Intestine-Chip.

### Example 10

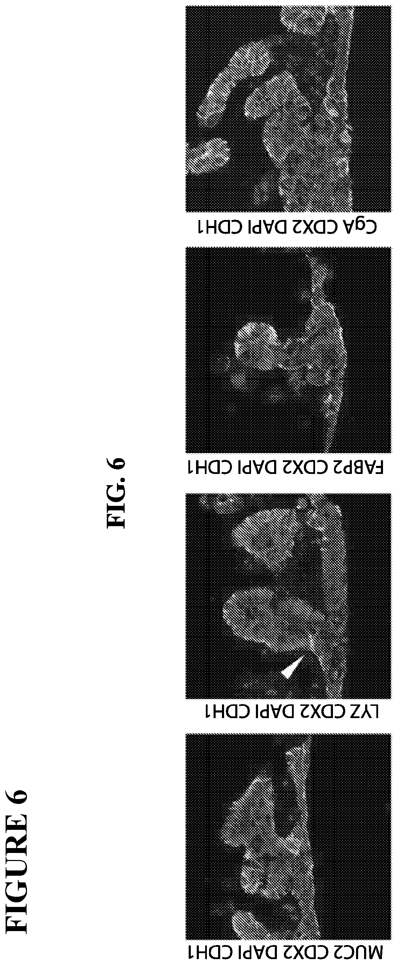

To demonstrate that the intestinal epithelial cells in the Intestine-Chip were biologically responsive to exogenous stimuli and therefore suitable for modeling epithelial responses, the Inventors chose to examine the response to IFNγ, a cytokine known to be upregulated in IBD. As Caco2 cells are routinely used to assess intestinal epithelial responses and have previously been incorporated into Chips, the Inventors also included these in the Inventors' studies to directly compare the Intestine-Chip. Exposure to IFNγ at concentrations ranging from 10-1000ng/ml resulted in the robust phosphorylation of STAT1 in both cell types when added to the lower channel for one hour (Fig. 3a). Thus both cell types were incubated with 10ng/ml of IFNγ for 3 days and assessed for upregulation of indoleamine 2 3-dioxygenase (IDO1) and guanylate binding protein 1 (GBP1). These were chosen as they are both upregulated in response to IFNγ and are expressed in the inflamed intestinal epithelium of IBD patients. The Inventors validated the results in these previous reports and found that there was a significant increase in IDO1 and GBP1 in HIO-epithelial cells as compared to controls (Fig. 3b). In contrast, while there was significant upregulation of IDO1 and GBP1 in Caco2 cells, it was attenuated in comparison to the HIO-epithelial cells. The Inventors then looked for changes in expression of various genes that are specific to Paneth and goblet cells and found that while there no differences in *lysozyme* and *Reg3*γ there was significant upregulation of the antimicrobial, *PLA2G2a* and the mucin, *Muc4* (Fig. 3c). Although the Inventors did observe immunopositive intestinal epithelial subtypes (Fig. 6a) in Chips containing Caco2 cells which is in agreement a previous study, the Inventors did not observe any significant upregulation of the aforementioned genes. These findings suggest that HIO-derived intestinal epithelial cells may be a more suitable models in which to predict intestinal epithelial responses.

### Example 11

To conclude, the Intestine-Chip combines the strengths of intestinal organoid and those of small microengineering technologies. The intestinal organoid component permits the incorporation of biologically responsive intestinal epithelial cells from almost any individual/patient into the system. Given that previous studies have shown that various immune cell types and microbes can be incorporated in Chips, the microengineering component allows for an examination of how an almost unlimited combination of cytokines, microbes and immune cells affects the functioning of intestinal epithelial cells in a patient specific manner. The Chips further allows the establishment and control of a more physiologically relevant microenvironment shown to improve cell function in vitro. Such an approach would be highly applicable in the gastrointestinal field and will most likely have major implications for personalized medicine.

### Example 12

### Colonic cells

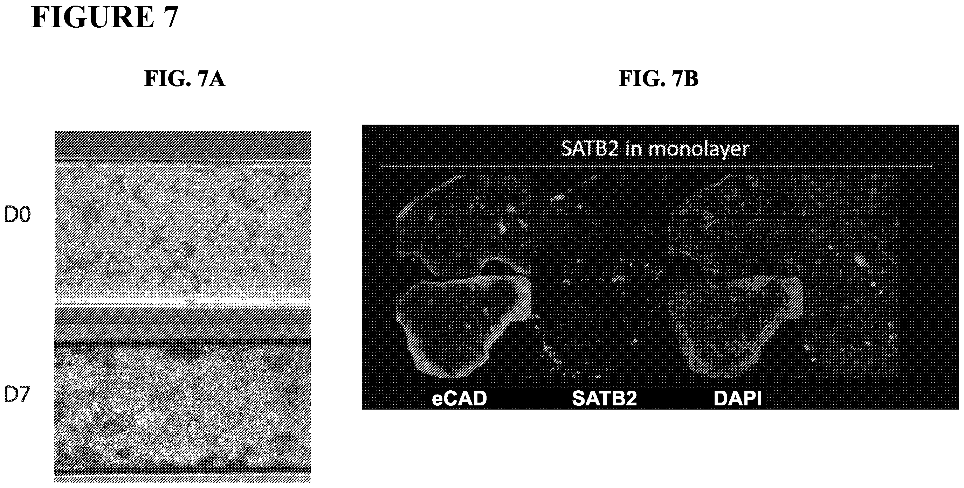

Definitive endoderm can be generated from iPSCs, including LCL and blood cell deried iPSCs by the aforementioned methods. This includes generation of definitive endoderm by culturing iPSCs in the presence of Activin A and Wnt3A, differentiation into hindgut by culturing definitive endoderm in the presence of FGF and either Wnt3A or CHIR99021, collection of epithelial spheres or epithelial tubes. For human intestinal organoids (HIOs), suspension of epithelial spheres or epithelial tubes in Matrigel, and culturing in the presence of CHIR99021, noggin and EGF. These intestinal cells can express one or more of PDX1, GATA4, DEFA5. For HCOs collection of epithelial spheres or epithelial tubes, suspension of epithelial spheres or epithelial tubes in Matrigel, is followed by culturing in the presence of EGF and BMP. These colonic cells can express one or more of SATB2, MUC5B, MUC2, INSL5, HOXA13, CKB, FXYD3, and HOXB13. Developmentally, posterior patterning of human definitive endoderm is dependent on BMP signaling and in vitro, this can be exploited to generate HCOs. Inhibition of BMP abrogates the ability of WNT and FGF to promote a posterior endoderm fate, thereby shunting towards HCO development and away from HIO formation. HCOs can be seeded onto microfluidic chips (Figure 7a). Figure 7b shows colonic SATB2 staining in a monolayer of cells that were seeded onto a microfluidic chip.

### Example 13

### Generation of VEO IBD patient lines

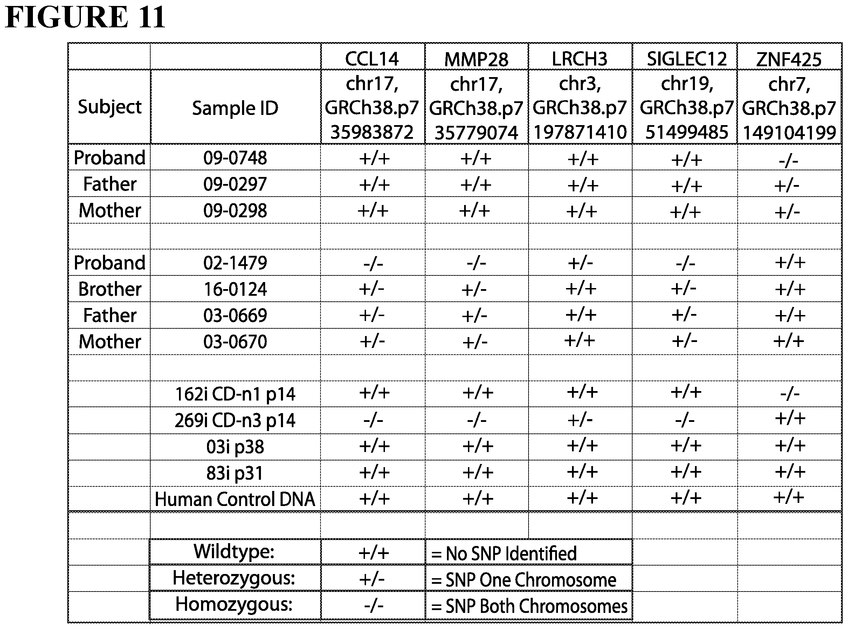

In accordance with various embodiments, iPSCs from very early onset (VEO) IBD patients were generated from blood cells. The Inventors obtained DNA from their patients and some of their siblings/parents and verified that in the corresponding iPSC lines (269i and 162i) that the variants of interest were persistent in iPSC lines. Genes listed in Figure 11 including generation of iPSCs from subjects possessing mutations in CCL14, MMP28, LRCH3, SIGLEC12, ZNF425. The 03i and 83i were generated from control lines. Details are shown in Figure 11.

### Example 14

### Transwell

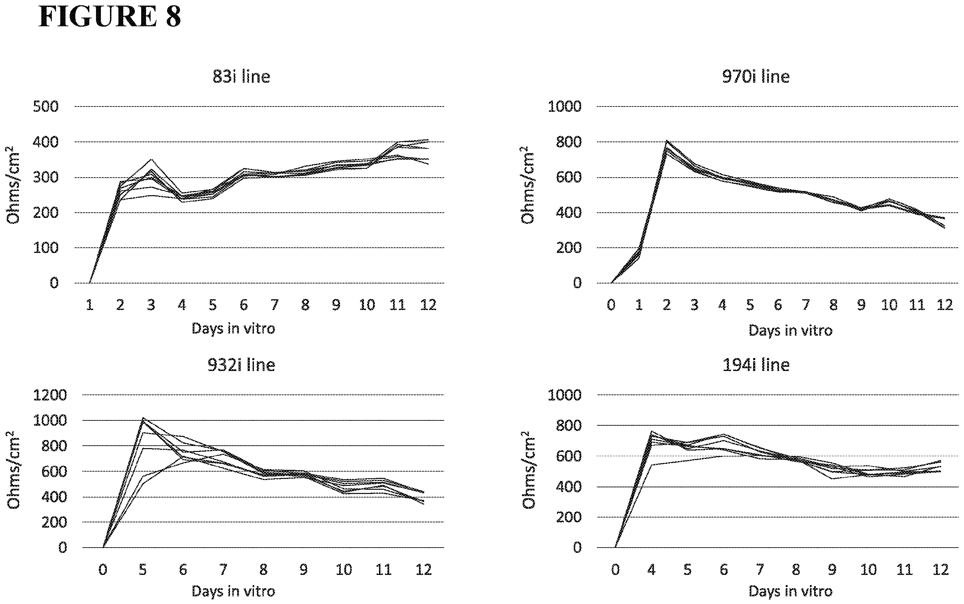

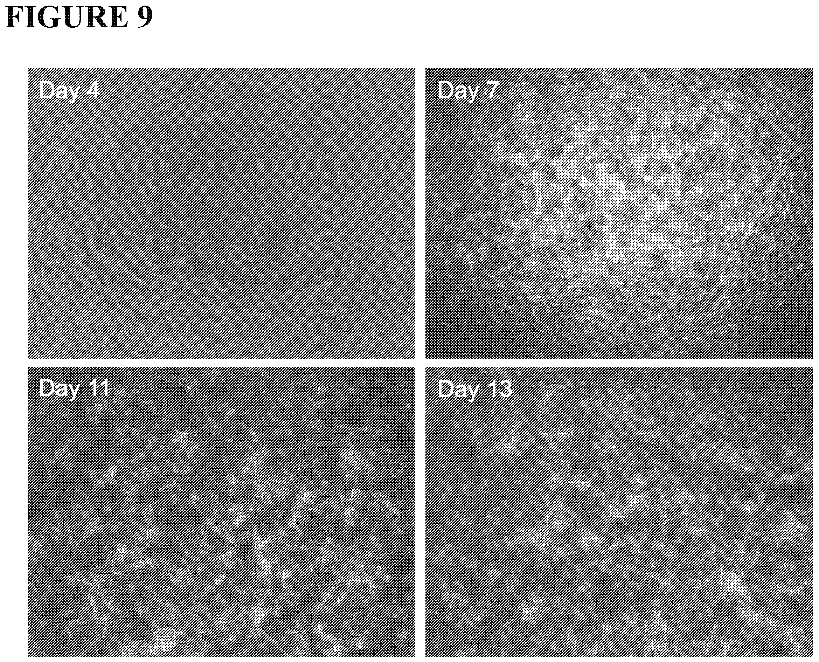

HIOs and HCOs can be evaluated in other apparatuses including transwell systems. HIOs, for example, allow for permeability measurements. Figure 8 depicts 4 different lines that were seeded on transwells and TEER was measured for 12 days. Each line represents an individual transwell and there were 8 transwell measured in each group. Figure 9 shows images of 83i cells when they grow over the various timepoints.

### Example 15

### Cryopreservation

One restriction on the use of intestinal enteroid cells derived from human iPS cell lines is that these cells need to be used during a certain time period for producing viable and reproducible microfluidic intestinal chips. However, during the development of the present inventions, methods and conditions were developed for using multiple aliquots (i.e. duplicate samples) of the same human intestinal enteroid cells in experiments separated by long time periods from the first experiment using these cells. Alternatively, intestinal enteroid cells derived from human iPS cell lines may be stored long term.

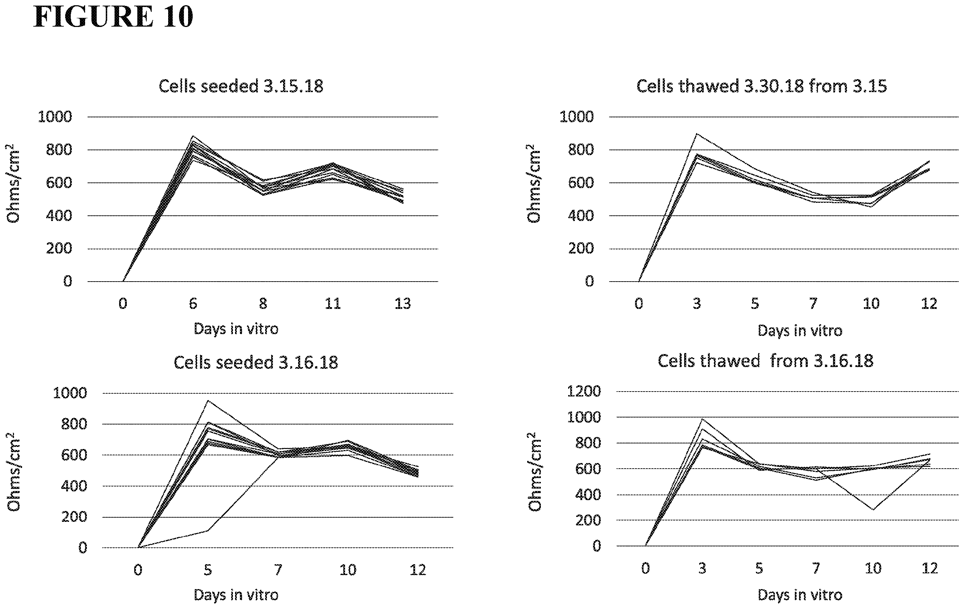

As an exemplary freezing method, iPS cells are cultured and differentiated into intestinal organoid cells then selected as described above. After the cells are selected for the desired subpopulation of cells, they are re-suspended in Cryostor in a sterial cryogenic vial/tube. Cryostor refers to a defined cryopreservation medium, as examples, CryoStor^{®} CS10 (serum-free, animal component-free, and defined cryopreservation medium containing 10% dimethyl sulfoxide (DMSO), 5% DMASO in CryoStor^{®} CS5 or 2% DMSO in CryoStor^{®}CS2, obtained from Stem Cell Technolgies. Cryogenic vials containing intestinal iPS cells are then frozen and stored in a liquid nitrogen tank. These thawed cells were also placed (seeded) in trans-wells producing viable cultures that grew well as shown in Figure 10

The various methods and techniques described above provide a number of ways to carry out the invention. Of course, it is to be understood that not necessarily all objectives or advantages described may be achieved in accordance with any particular embodiment described herein. Thus, for example, those skilled in the art will recognize that the methods can be performed in a manner that achieves or optimizes one advantage or group of advantages as taught herein without necessarily achieving other objectives or advantages as may be taught or suggested herein. A variety of advantageous and disadvantageous alternatives are mentioned herein. It is to be understood that some preferred embodiments specifically include one, another, or several advantageous features, while others specifically exclude one, another, or several disadvantageous features, while still others specifically mitigate a present disadvantageous feature by inclusion of one, another, or several advantageous features.

Furthermore, the skilled artisan will recognize the applicability of various features from different embodiments. Similarly, the various elements, features and steps discussed above, as well as other known equivalents for each such element, feature or step, can be mixed and matched by one of ordinary skill in this art to perform methods in accordance with principles described herein. Among the various elements, features, and steps some will be specifically included and others specifically excluded in diverse embodiments.

Although the invention has been disclosed in the context of certain embodiments and examples, it will be understood by those skilled in the art that the embodiments of the invention extend beyond the specifically disclosed embodiments to other alternative embodiments and/or uses and modifications and equivalents thereof.

Many variations and alternative elements have been disclosed in embodiments of the present invention. Still further variations and alternate elements will be apparent to one of skill in the art. Among these variations, without limitation, are the compositions and methods related to induced pluripotent stem cells (iPSCs), differentiated iPSCs including intestinal and colonic cells, methods and compositions related to use of the aforementioned compositions, techniques and composition and use of solutions used therein, and the particular use of the products created through the teachings of the invention. Various embodiments of the invention can specifically include or exclude any of these variations or elements.

In some embodiments, the numbers expressing quantities of ingredients, properties such as concentration, reaction conditions, and so forth, used to describe and claim certain embodiments of the invention are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The numerical values presented in some embodiments of the invention may contain certain errors necessarily resulting from the standard deviation found in their respective testing measurements.

In some embodiments, the terms "a" and "an" and "the" and similar references used in the context of describing a particular embodiment of the invention (especially in the context of certain of the following claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

Preferred embodiments of this invention are described herein, including the best mode known to the inventor for carrying out the invention. Variations on those preferred embodiments will become apparent to those of ordinary skill in the art upon reading the foregoing description. It is contemplated that skilled artisans can employ such variations as appropriate, and the invention can be practiced otherwise than specifically described herein. Accordingly, many embodiments of this invention include all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A method of generating colonic cells, comprising:
generation of definitive endoderm by culturing iPSCs in the presence of Activin A and Wnt3A, and increasing concentrations of fetal bovine serum (FBS) over time of 0.2%, 0.2%-0.5%, and 1%-2%, on each of days 1, 2, and 3 respectively;
differentiation into hindgut by culturing definitive endoderm in the presence of FGF4 and either Wnt3A or CHIR99021;
collection of epithelial spheres or epithelial tubes;
suspension of epithelial spheres or epithelial tubes in Matrigel; and
culturing in the presence of EGF and BMP to generate colonic cells.

2. The method of claim 1, wherein the colonic cells express one or more of: SATB2, MUC5B, MUC2, INSL5, HOXA13, CKB, FXYD3, and HOXB13.

3. The method of claim 1, wherein the iPSCs are reprogrammed cells from whole or peripheral blood, and wherein the iPSCs are reprogrammed from a non B-Cell, non T-cell component of blood.

4. The method of claim 1, wherein the iPSCs are reprogrammed lymphoblastoid B-cell derived induced pluripotent stem cells (LCL-iPSCs).

5. The method of claim 1, wherein the iPSCs are reprogrammed cells obtained from a subject afflicted with an inflammatory bowel disease and/or condition.

6. The method of claim 5, wherein the inflammatory bowel disease and/or condition is early onset.

7. The method of claim 5, wherein the inflammatory bowel disease and/or condition is very early onset.

8. The method of claim 1, wherein the concentration of Activin A is 25-50 ng/ml, 50-75 ng/ml, 100-125ng/ml, or 125-150 ng/ml, and the concentration of Wnt3A is 25-50 ng/ml, 50-75 ng/ml, 100-125ng/ml, or 125-150 ng/ml, optionally
wherein the concentration of Activin A is 100ng/ml, the concentration of Wnt3A is 25ng/ml, and the concentrations of FBS are increased over time, 0.2%, 0.5%, and 2% on each of days 1, 2, and 3, respectively, for generation of definitive endoderm.

9. The method of claim 1 or claim 8, wherein differentiation into hindgut comprises culturing the definitive endoderm in 0.2%-0.5%, 0.5%-1%, or 1%-2% FBS, 100-250 ng/ml, 250-500ng/ml, or 500 ng/ml or more FGF4 and either 100-250 ng/ml, 250-500ng/ml, or 500 ng/ml Wnt3A or 0.5-1 µM, 1-1.5 µM, 1.5-2 µM, or 2 µM or more CHIR99021, or both for 1, 2, 3, 4 or more days

10. The method of claim 9, wherein the concentration of FBS is 2% and the concentration of FGF4 is 500ng/ml, for differentiation into hindgut, and/or
wherein the concentration of Wnt3A is 500ng/ml and/or the concentration of CHIR99021 is 3 µM, for differentiation into hindgut.

11. The method of any one of claims 1, 8, 9, or 10, wherein the concentration of EGF is 50-100 ng/ml, 100-250 ng/ml, 250-500ng/ml, or 500 ng/ml or more, and the concentration of BMP is 50-100 ng/ml, 100-250 ng/ml, 250-500ng/ml, or 500 ng/ml or more, optionally
wherein the concentration of EGF is 100ng/ml and the concentration of BMP is 100ng/ml.

12. The method of any one of claims 1, 8, 9, 10, or 11, wherein the definitive endoderm is cultured in media comprising FGF4, Wnt3A, and CHIR99021, for differentiation into hindgut.

13. The method of claim 12, wherein the definitive endoderm is cultured in media comprising 500ng/ml FGF4, 500ng/ml Wnt3A, and 3 µM CHIR99021, for differentiation into hindgut.

## Patentansprüche

1. Verfahren zum Erzeugen von Kolonzellen, umfassend:
Erzeugung von definitivem Entoderm durch Kultivieren von iPSCs in Gegenwart von Activin A und Wnt3A und Erhöhen von Konzentrationen von fötalem Rinderserum (FBS) im Laufe der Zeit von 0,2 %, 0,2 %-0,5 % und 1 %-2 % an jedem von Tag 1, 2 bzw. 3;
Differenzierung in Enddarm durch Kultivieren von definitivem Entoderm in Gegenwart von FGF4 und entweder Wnt3A oder CHIR99021;
Sammlung von Epithelkugeln oder Epithelröhren;
Suspension von Epithelkugeln oder Epithelröhren in Matrigel; und
Kultivieren in Gegenwart von EGF und BMP, um Kolonzellen zu erzeugen.

2. Verfahren nach Anspruch 1, wobei die Kolonzellen eines oder mehrere von Folgenden exprimieren: SATB2, MUC5B, MUC2, INSL5, HOXA13, CKB, FXYD3 und HOXB13.

3. Verfahren nach Anspruch 1, wobei die iPSCs umprogrammierte Zellen aus Vollblut oder peripherem Blut sind und wobei die iPSCs aus einer Nicht-B-Zell-, Nicht-T-Zell-Blutkomponente umprogrammiert sind.

4. Verfahren nach Anspruch 1, wobei die iPSCs umprogrammierte aus lymphoblastoiden B-Zellen abgeleitete induzierte pluripotente Stammzellen (LCL-iPSCs) sind.

5. Verfahren nach Anspruch 1, wobei die iPSCs umprogrammierte Zellen sind, die von einem Subjekt gewonnen wurden, das an einer entzündlichen Darmerkrankung und/oder -krankheit leidet.

6. Verfahren nach Anspruch 5, wobei die entzündliche Darmerkrankung und/oder -krankheit frühmanifest ist.

7. Verfahren nach Anspruch 5, wobei die entzündliche Darmerkrankung und/oder -krankheit sehr frühmanifest ist.

8. Verfahren nach Anspruch 1, wobei die Konzentration von Activin A 25-50 ng/ml, 50-75 ng/ml, 100-125 ng/ml oder 125-150 ng/ml beträgt und die Konzentration von Wnt3A 25-50 ng/ml, 50-75 ng/ml, 100-125 ng/ml oder 125-150 ng/ml beträgt, optional
wobei zur Erzeugung von definitivem Entoderm die Konzentration von Activin A 100 ng/ml beträgt, die Konzentration von Wnt3A 25 ng/ml beträgt und die Konzentrationen von FBS im Laufe der Zeit, 0,2 %, 0,5 % und 2 % an jedem von Tag 1, 2 bzw. 3, erhöht werden.

9. Verfahren nach Anspruch 1 oder Anspruch 8, wobei die Differenzierung in Enddarm Kultivieren des definitiven Entoderms in 0,2 %-0,5 %, 0,5 %-1 % oder 1 %-2 % FBS, 100-250 ng/ml, 250-500 ng/ml oder 500 ng/ml oder mehr FGF4 und entweder 100-250 ng/ml, 250-500 ng/ml oder 500 ng/ml Wnt3A oder 0,5-1 µM, 1-1,5 µM, 1,5-2 µM oder 2 µM oder mehr CHIR99021 oder beidem für 1, 2, 3, 4 oder mehr Tage umfasst.

10. Verfahren nach Anspruch 9, wobei zur Differenzierung in Enddarm die Konzentration von FBS 2 % beträgt und die Konzentration von FGF4 500 ng/ml beträgt und/oder
wobei zur Differenzierung in Enddarm die Konzentration von Wnt3A 500 ng/ml beträgt und/oder die Konzentration von CHIR99021 3 µM beträgt.

11. Verfahren nach einem der Ansprüche 1, 8, 9 oder 10, wobei die Konzentration von EGF 50-100 ng/ml, 100-250 ng/ml, 250-500 ng/ml oder 500 ng/ml oder mehr beträgt und die Konzentration von BMP 50-100 ng/ml, 100-250 ng/ml, 250-500 ng/ml oder 500 ng/ml oder mehr beträgt, optional
wobei die Konzentration von EGF 100 ng/ml beträgt und die Konzentration von BMP 100 ng/ml beträgt.

12. Verfahren nach einem der Ansprüche 1, 8, 9, 10 oder 11, wobei das definitive Entoderm zur Differenzierung in Enddarm in Medien kultiviert wird, die FGF4, Wnt3A und CHIR99021 umfassen.

13. Verfahren nach Anspruch 12, wobei das definitive Entoderm zur Differenzierung in Enddarm in Medien kultiviert wird, die 500 ng/ml FGF4, 500 ng/ml Wnt3A und 3 µM CHIR99021 umfassen.

## Revendications

1. Procédé de génération de cellules du côlon, comprenant :
la génération d'endoderme définitif en cultivant des iPSC en présence d'activine A et de Wnt3A et en augmentant les concentrations de sérum bovin foetal (FBS) au fil du temps de 0,2 %, 0,2 % à 0,5 % et 1 % à 2 %, chacun des jours 1, 2 et 3 respectivement ;
la différenciation en intestin postérieur par culture de l'endoderme définitif en présence de FGF4 et soit de Wnt3A soit de CHIR99021 ;
la collecte de sphères épithéliales ou de tubes épithéliaux ;
la suspension de sphères épithéliales ou de tubes épithéliaux dans du Matrigel ; et
la culture en présence d'EGF et de BMP pour générer des cellules de côlon.

2. Procédé selon la revendication 1, lesdites cellules du côlon exprimant un ou plusieurs des suivants : SATB2, MUC5B, MUC2, INSL5, HOXA13, CKB, FXYD3 et HOXB13.

3. Procédé selon la revendication 1, lesdites iPSC étant des cellules reprogrammées provenant de sang total ou périphérique, et lesdites iPSC étant reprogrammées à partir d'un composant du sang non-lymphocyte B, non-lymphocyte T.

4. Procédé selon la revendication 1, lesdites iPSC étant des cellules souches pluripotentes induites dérivées de lymphocytes B lymphoblastoïdes (LCL-iPSC) reprogrammées.

5. Procédé selon la revendication 1, lesdites iPSC étant des cellules reprogrammées prélevées chez un sujet atteint d'une maladie et/ou d'un état inflammatoire de l'intestin.

6. Procédé selon la revendication 5, ladite maladie et/ou ledit état inflammatoire de l'intestin présentant une apparition précoce.

7. Procédé selon la revendication 5, ladite maladie et/ou ledit état inflammatoire de l'intestin présentant une apparition très précoce.

8. Procédé selon la revendication 1, ladite concentration d'activine A étant de 25 à 50 ng/ml, 50 à 75 ng/ml, 100 à 125 ng/ml ou 125 à 150 ng/ml, et ladite concentration de Wnt3A étant de 25 à 50 ng/ml, 50 à 75 ng/ml, 100 à 125 ng/ml ou 125 à 150 ng/ml, éventuellement
ladite concentration d'activine A étant de 100 ng/ml, ladite concentration de Wnt3A étant de 25 ng/ml, et lesdites concentrations de FBS étant accrues au fil du temps, de 0,2 %, 0,5 % et 2 % chacun des jours 1, 2 et 3, respectivement, pour la génération de l'endoderme définitif.

9. Procédé selon la revendication 1 ou la revendication 8, ladite différenciation en intestin postérieur comprenant la culture de l'endoderme définitif dans 0,2 % à 0,5 %, 0,5 % à 1 %, ou 1 % à 2 % de FBS, 100 à 250 ng/ml, 250 à 500 ng/ml, ou 500 ng/ml ou plus de FGF4 et soit 100 à 250 ng/ml, 250 à 500 ng/ml ou 500 ng/ml de Wnt3A ou 0,5 à 1 µM, 1 à 1,5 µM, 1,5 à 2 µM, ou 2 µM ou plus de CHIR99021, ou les deux pendant 1, 2, 3, 4 jours ou plus.

10. Procédé selon la revendication 9, ladite concentration de FBS étant de 2 % et ladite concentration de FGF4 étant de 500 ng/ml, pour la différenciation en intestin postérieur, et/ou
ladite concentration de Wnt3A étant de 500 ng/ml et/ou ladite concentration de CHIR99021 étant de 3 µM, pour la différenciation en intestin postérieur.

11. Procédé selon l'une quelconque des revendications 1, 8, 9 ou 10, ladite concentration d'EGF étant de 50 à 100 ng/ml, 100 à 250 ng/ml, 250 à 500 ng/ml, ou 500 ng/ml ou plus, et ladite concentration de BMP étant de 50 à 100 ng/ml, 100 à 250 ng/ml, 250 à 500 ng/ml, ou 500 ng/ml ou plus, éventuellement
ladite concentration d'EGF étant de 100 ng/ml et ladite concentration de BMP étant de 100 ng/ml.

12. Procédé selon l'une quelconque des revendications 1, 8, 9, 10 ou 11, ledit endoderme définitif étant cultivé dans des milieux comprenant FGF4, Wnt3A et CHIR99021, pour la différenciation en intestin postérieur.

13. Procédé selon la revendication 12, ledit endoderme définitif étant cultivé dans un milieu comprenant 500 ng/ml de FGF4, 500 ng/ml de Wnt3A et 3 µM de CHIR99021, pour la différenciation en intestin postérieur.
